# EUROPÄISCHE PATENTSCHRIFT

(11) **EP 0 869 116 B1**
(45) Veröffentlichungstag und Bekanntmachung des Hinweises auf die Patenterteilung: **12.12.2001**
(21) Anmeldenummer: 98105453.9
(22) Anmeldetag: 26.03.1998
(51) Int. Cl.: C07C 279/22, A61K 31/155

(54) **Ortho-substituierte Benzoylguanidine, Verfahren zu ihrer Herstellung, ihre Verwendung als Medikament oder Diagnostikum sowie sie enthaltendes Medikament**
Ortho-substituted benzoylguanidines, process for their preparation, their use as medicament or diagnostic agent as well as medicaments containing them
Benzoylguanidines ortho-substituées, procédé pour leur préparation, leur utilisation comme médicament ou agent diagnostique ainsi que médicaments les contenant

(30) Priorität: 01.04.1997 DE 19713427
(43) Veröffentlichungstag der Anmeldung: 07.10.1998
(73) Patentinhaber: HOECHST AKTIENGESELLSCHAFT, 65929 Frankfurt am Main (DE)
(72) Erfinder: Weichert, Andreas, Dr., 63329 Egelsbach (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Schwark, Jan-Robert, Dr., 65929 Frankfurt (DE); Albus, Udo, Dr., 61197 Florstadt (DE); Scholz, Wolfgang, Dr., 65760 Eschborn (DE)

(56) Entgegenhaltungen:
- EP-A- 0 640 588
- EP-A- 0 704 431
- EP-A- 0 814 077

## Beschreibung

Die Erfindung betrifft Benzoylguanidine der Formel I 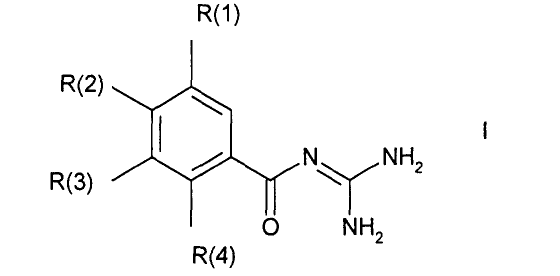 worin bedeuten:
- R(1): CF₃;
einer der Substituenten R(2) und R(3) Wasserstoff;
und der jeweils andere Substituent R(2) oder R(3) -C(OH)(CH₃)-CH₂OH, -CH(CH₃)-CH₂OH oder -C(OH)(CH₃)₂;
- R(4): Methyl, Methoxy, Cl oder CF₃;
sowie deren pharmazeutisch verträgliche Salze.

Bevorzugt sind Verbindungen der Formel I, in denen bedeuten
- R(1): CF₃;
einer der Substituenten R(2) und R(3) Wasserstoff;
und der jeweils andere Substituent R(2) oder R(3) -C(OH)(CH₃)-CH₂OH, -CH(CH₃)-CH₂OH oder -C(OH)(CH₃)₂;
- R(4): Methyl;
sowie deren pharmazeutisch verträgliche Salze.

Besonders bevorzugt sind Verbindungen der Formel I, in denen bedeuten:
- R(1): CF₃;
einer der Substituenten R(2) und R(3) Wasserstoff;
und der jeweils andere Substituent R(2) oder R(3) -CH(CH₃)-CH₂OH;
- R(4): Methyl;
sowie deren pharmazeutisch verträgliche Salze.

Ganz besonders bevorzugt ist die Verbindung der Formel I,
in der bedeuten:
- R(1): CF₃;
- R(2): -CH(CH₃)-CH₂OH;
- R(3): Wasserstoff;
- R(4): Methyl;
sowie deren pharmazeutisch verträgliche Salze.

Enthält einer der Substituenten R(2) oder R(3) ein Asymmetriezentrum, so können diese sowohl S als auch R konfiguriert sein. Die Verbindungen können als optische Isomere, als Diastereomere, als Racemate oder als Gemische derselben vorliegen.

Die Erfindung betrifft außerdem ein Verfahren zur Herstellung einer Verbindung der Formel I, das dadurch gekennzeichnet ist, daß man eine Verbindung der Formel II 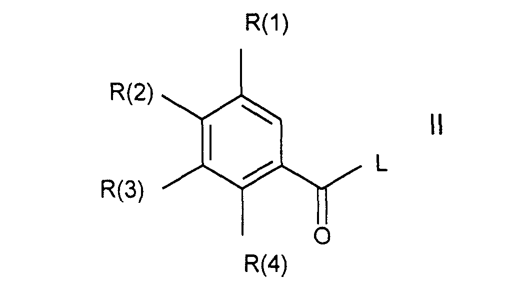 worin R(1) bis R(4) die angegebene Bedeutung besitzen und L für eine leicht nucleophil substituierbare Fluchtgruppe steht, mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-Derivaten (Formel II, L = OH) herstellen, wie die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung der Substituenten in 2-, 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Den erfindungsgemäßen Verbindungen der Formel I ähnliche Verbindungen sind aus der Europäischen Offenlegungsschrift 640 588 (HOE 93/F 254) bekannt, unter deren allgemeine Formel die erfindungsgemäßen Verbindungen fallen. Sie zeichnen sich aber gegenüber den bekannten Verbindungen durch eine ungewöhnlich und überraschend viel höhere Wirksamkeit in der Inhibition des Na⁺/H⁺-Austauschs aus, verbunden mit einer hervorragende Wasserlöslichkeit.

Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheiten wichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei OrganTransplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, daß Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, daß Verbindungen der Formel I bezüglich der Beeinflussung der Serumlipidspiegel wertvolle therapeutisch verwertbare Eigenschaften zeigen. So erniedrigen sie signifikant die erhöhte Serum Konzentration von LDL und VLDL, wie sie beispielweise durch erhöhte dietätische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einen wirksamen Schutz gegen durch Stoffwechselanomalien induzierte Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten, eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:
- MeOH: Methanol
- DMF: N,N-Dimethylformamid
- RT: Raumtemperatur
- EE: Ethylacetat (EtOAc)
- El: electron impact
- ES: Elektrospray-lonisierung
- mp: Schmelzpunkt
- THF: Tetrahydrofuran
- eq: Äquivalent

### Experimenteller Teil

### Allgemeine Vorschrift zur Herstellung von Benzoyl-guanidinen (I) Variante A: aus Benzoesäuren (II, L = OH)

1.0 eq. des Benzoesäurederivates der Formel II löst bzw. suspendiert man in wasserfreiem THF ( 5 ml / mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCI auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Sieden erhitzt (typische Reaktionszeit 2 bis 5 h). Das Lösungsmittel wird unter vermindertem Druck (Rotationsverdampfer) abdestilliert, in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel: 4-(1'-Hydroxy-2'-propyl)-2-methyl-5-trifluormethyl-benzoylguanidinhydrochlorid,

farblose Kristalle, Zersetzung ab 100°C, MS (ES): M⁺ + H = 304

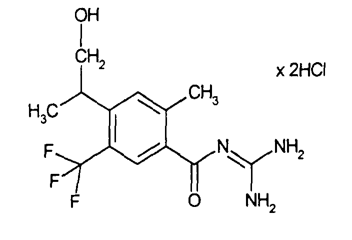

### Syntheseweg:

a) 4-Hydroxy-2-chlor-5-iod-benzoesäuremethylester
   aus 4-Hydroxy-2-chlor-benzoesäuremethylester durch Olah-lodierung mit 1 eq N-lod-Succinimid in 5 eq Trifluormethansulfonsäure bei RT für 24 h, farblose Kristalle,
   mp. 188 - 89°C, MS (El): M⁺ = 312.
b) 4-Benzyloxy-2-chlor-5-iod-benzoesäuremethylester
   aus 1 a) durch Reaktion mit 1 eq Benzylbromid in Gegenwart von 1.5 eq Kaliumcarbonat in absolutem DMF bei RT. Wäßrige Aufarbeitung gefolgt von Umkristallisation aus i-Propanol ergibt
   farblose Kristalle,
   mp. 107 - 08°C, MS(ES): M⁺ +H = 403.
c) 4-Benzyloxy-2-chlor-5-trifluormethyl-benzoesäuremethylester
   aus 1 b) Umsetzung mit 1 eq Kaliumtrifluoracetat und 1.1 eq Kupfer(I)iodid in DMF unter Rückfluß. Säulenchromatographische Aufarbeitung ergibt
   farblosen Feststoff,
   mp. 127 - 28°C, MS (ES): M⁺ +H = 345.
d) 4-Benzyloxy-2-methyl-5-trifluormethyl-benzoesäuremethylester
   aus 1 c) durch cross-coupling mit 5 eq Methylzinkchlorid in THF / DMF unter Rückfluß in Gegenwart von katalytischem 0.1 eq Palladium(II)acetat, 0.2 eq Triphenylphosphin und 0.11 eq Kupfer(I)iodid, wäßrige Aufarbeitung, Extraktion mit Essigester und anschließende Säulenchromatographie an Kieselgel liefert farblosen Feststoff,
   mp. 105°C, MS (ES): M ⁺ + H = 325.
e) 4-Hydroxy-2-methyl-5-trifluormethyl-benzoesäuremethylester
   aus 1 d) durch Hydrogenolyse in Gegenwart von Palladium/Kohle (10%) in Methanol bei RT. Zusetzen von Cyclohexan bewirkt Kristallisation eines farblosen Feststoffs,
   mp. 162 - 63°C, MS (ES): M⁺ +H = 235.
f) 4-Trifluormethansulfonyloxy-2-methyl-5-trifluormethyl-benzoesäuremethylester
   aus 1 e) durch Umsetzung mit 1.1 eq Trifluormethansulfonsäureanhydrid in Gegenwart von 1.1 eq Triethylamin in absolutem Methylenchlorid bei 0°C. Säulenchromatographie ergibt
   amorphen Feststoff.
g) 4-lsopropenyl-2-methyl-5-trifluormethyl-benzoesäuremethylester
   aus 1 f) durch cross-coupling mit 5 Äquivalenten Isopropenylzinkchlorid in THF/DMF unter Rückfluß in Gegenwart von katalytischem 0.1 eq Palladium(II)acetat, 0.2 eq Triphenylphosphin und 0.11 eq Kupfer(I)iodid, wäßrige Aufarbeitung, Extraktion mit Essigester und anschließende Säulenchromatographie an Kieselgel liefert ein
   farbloses Öl, MS (ES): M⁺ +H = 259.
h) 4-(1'-Hydroxy-2'-propyl)-2-methyl-5-trifluormethyl-benzoesäure-methylester
   aus 1 g) durch Hydroborierung mit 1.05 eq Boran-Tetrahydrofuran-Komplex und anschließender oxidativer Reaktionsführung (alkalisches Wasserstoffperoxid). Nach wäßriger Aufarbeitung und säulenchromatogrphischer Reinigung fällt ein farbloses Öl
   an, MS (ES): M⁺ +H = 277.
i) 4-(1'-Hydroxy-2'-propyl)-2-methyl-5-trifluormethyl-benzoylguanidin-hydrochlorid
aus 1 h) nach allgemeiner Vorschrift, Variante B.

### Pharmakologische Daten:

Inhibition des Na⁺ /H ⁺ -Exchangers von Kaninchenerythrocyten Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na ⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

Inhibition des Na⁺/H⁺-Exchangers:

| | |
|---|---|
| Beispiel 1 | IC₅₀ (mol /l) 0.0012 x 10⁻⁶ |

## Patentansprüche

1. Benzoylguanidine der Formel I 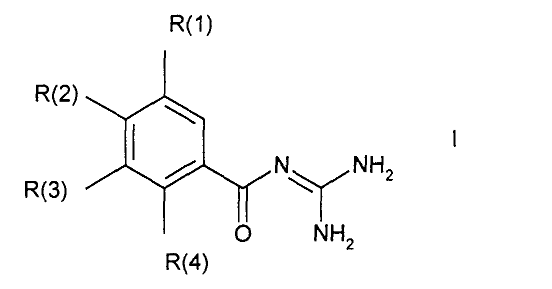 worin bedeuten:
R(1) CF₃;
einer der Substituenten R(2) und R(3)
Wasserstoff;
und der jeweils andere Substituent R(2) oder R(3)
-C(OH)(CH₃)-CH₂OH, -CH(CH₃)-CH₂OH oder -C(OH)(CH₃)₂;
R(4) Methyl, Methoxy, Cl oder CF₃;
sowie deren pharmazeutisch verträgliche Salze.

2. Verbindungen der Formel I nach Anspruch 1, in der bedeuten
R(1) CF₃;
einer der Substituenten R(2) und R(3)
Wasserstoff;
und der jeweils andere Substituent R(2) oder R(3)
-C(OH)(CH₃)-CH₂OH, -CH(CH₃)-CH₂OH oder -C(OH)(CH₃)₂;
R(4) Methyl.

3. Verbindungen der Formel I nach Ansprüchen 1 oder 2, in der bedeuten:
R(1) CF₃;
einer der Substituenten R(2) und R(3)
Wasserstoff;
und der jeweils andere Substituent R(2) oder R(3)
-CH(CH₃)-CH₂OH;
R(4) Methyl.

4. Verbindung der Formel I nach einem der Ansprüche 1 bis 3,
in der bedeuten:
R(1) CF₃;
R(2) -CH(CH₃)-CH₂OH;
R(3) Wasserstoff;
R(4) Methyl;
sowie deren pharmazeutisch verträgliche Salze.

5. Verfahren zur Herstellung einer Verbindung der Formel I nach Anspruch 1, **dadurch gekennzeichnet, daß** man
eine Verbindung der Formel II 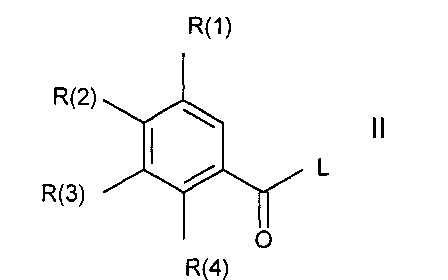 worin R(1) bis R(4) die angegebene Bedeutung besitzen und L für eine leicht nucleophil substituierbare Fluchtgruppe steht, mit Guanidin umsetzt.

6. Verwendung einer Verbindung I nach Anspruch 1 zur Herstellung eines Medikaments zur Behandlung oder Prophylaxe von durch ischämische Zustände bewirkten Krankheiten.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie.

16. Verwendung einer Verbindung I nach Anspruch 1 zur Herstellung eines Medikaments zur Behandlung oder Prophylaxe von Störungen des Fettstoffwechsels.

17. Arzneimittel, **gekennzeichnet durch** einen wirksamen Gehalt einer Verbindung der Formel I nach Ansprüchen 1 bis 4.

## Claims

1. A benzoylguanidine of the formula I 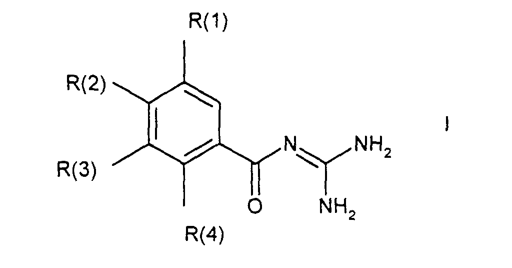 in which:
R(1) is CF₃;
one of the substituents R(2) and R(3)
is hydrogen;
and the other substituent R(2) or R(3) in each case is -C(OH)(CH₃)-CH₂OH, -CH(CH₃)-CH₂OH or -C(OH)(CH₃)₂;
R(4) is methyl, methoxy, Cl or CF₃;
or its pharmaceutically tolerable salts.

2. A compound of the formula I as claimed in claim 1, in which
R(1) is CF₃;
one of the substituents R(2) and R(3)
is hydrogen;
and the other substituent R(2)or R(3) in each case
is -C(OH)(CH₃)-CH₂OH, -CH(CH₃)-CH₂OH or -C(OH)(CH₃)₂;
R(4) is methyl.

3. A compound of the formula I as claimed in claim 1 or 2, in which:
R(1) is CF₃;
one of the substituents R(2) and R(3)
is hydrogen;
and the other substituent R(2) or R(3) in each case
is -CH(CH₃)-CH₂OH;
R(4) is methyl.

4. A compound of the formula I as claimed in one of claims 1 to 3,
in which:
R(1) is CF₃;
R(2) is -CH(CH₃)-CH₂OH;
R(3) is hydrogen;
R(4) is methyl;
or its pharmaceutically tolerable salts.

5. A process for the preparation of a compound of the formula I as claimed in claim 1, which comprises reacting
a compound of the formula II 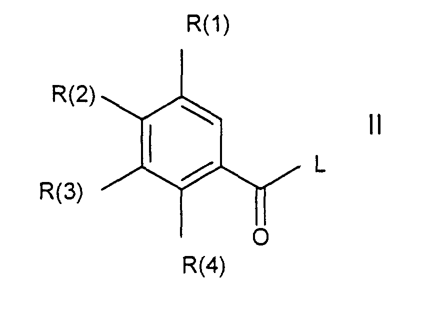 in which R(1) to R(4) have the meaning indicated and L is an easily nucleophilically substitutable leaving group, with guanidine.

6. The use of a compound I as claimed in claim 1 for the production of a medicament for the treatment or prophylaxis of illnesses caused by ischemic conditions.

7. The use of a compound I as claimed in claim 1 for the production of a medicament for the treatment or prophylaxis of cardiac infarct.

8. The use of a compound I as claimed in claim 1 for the production of a medicament for the treatment or prophylaxis of angina pectoris.

9. The use of a compound I as claimed in claim 1 for the production of a medicament for the treatment or prophylaxis of ischemic conditions of the heart.

10. The use of a compound I as claimed in claim 1 for the production of a medicament for the treatment or prophylaxis of ischemic conditions of the peripheral and central nervous system and of strokes.

11. The use of a compound I as claimed in claim 1 for the production of a medicament for the treatment or prophylaxis of ischemic conditions of peripheral organs and limbs.

12. The use of a compound I as claimed in claim 1 for the production of a medicament for the treatment of states of shock.

13. The use of a compound I as claimed in claim 1 for the production of a medicament for use in surgical operations and organ transplantation.

14. The use of a compound I as claimed in claim 1 for the production of a medicament for the preservation and storage of transplants for surgical measures.

15. The use of a compound I as claimed in claim 1 for the production of a medicament for the treatment of illnesses in which cell proliferation is a primary or secondary cause, and thus their use for the production of an antiatherosclerotic, of an agent against diabetic late complications, carcinomatous disorders, fibrotic disorders such as pulmonary fibrosis, hepatic fibrosis or renal fibrosis, prostate hyperplasia.

16. The use of a compound I as claimed in claim 1 for the production of a medicament for the treatment or prophylaxis of disorders of lipid metabolism.

17. A pharmaceutical which contains an efficacious amount of a compound of the formula I as claimed in claims 1 to 4.

## Revendications

1. Benzoylguanidines de formule I 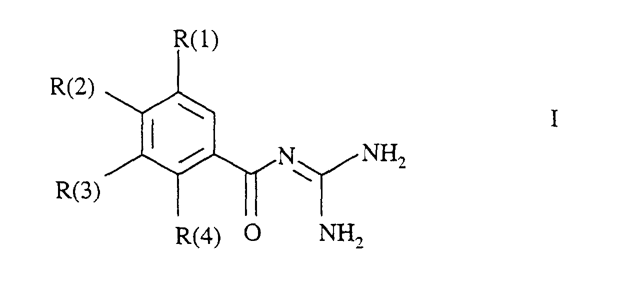 où :
R(1) représente CF₃;
l'un des substituants R(2) et R(3) représente l'hydrogène ;
et l'autre substituant R(2) ou R(3) représente
-C(OH)(CH₃)-CH₂OH, -CH(CH₃)-CH₂OH ou -C(OH)(CH₃)₂ ;
R(4) représente méthyle, méthoxy, Cl ou CF₃ ;
ainsi que leurs sels pharmaceutiquement acceptables.

2. Composés de formule I selon la revendication 1, où :
R(1) représente CF₃ ;
l'un des substituants R(2) et R(3) représente l'hydrogène ;
et l'autre substituant R(2) ou R(3) représente
-C(OH)(CH₃)-CH₂OH, -CH(CH₃)-CH₂OH ou -C(OH)(CH₃)₂;
R(4) représente méthyle.

3. Composés de formule I selon la revendication 1 ou 2 où :
R(1) représente CF₃ ;
l'un des substituants R(2) et R(3) représente l'hydrogène ;
et l'autre substituant R(2) ou R(3) représente -CH(CH₃)-CH₂OH ;
R(4) représente méthyle.

4. Composé de formule I selon l'une des revendications 1 à 3 où :
R(1) représente CF₃ ;
R(2) représente -CH(CH₃)-CH₂OH ;
R(3) représente l'hydrogène ;
R(4) représente méthyle ;
ainsi que ses sels pharmaceutiquement acceptables.

5. Procédé de préparation d'un composé de formule I selon la revendication 1, **caractérisé en ce que** l'on fait réagir avec la guanidine un composé de formule II 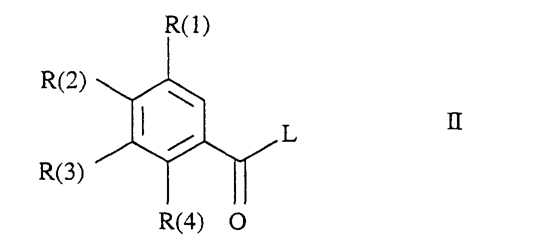 où R(1) à R(4) possèdent la signification indiquée et L représente un groupe partant aisément substituable par une substitution nucléophile.

6. Utilisation d'un composé I selon la revendication 1 pour la préparation d'un médicament pour le traitement ou la prophylaxie de maladies provoquées par des états ischémiques.

7. Utilisation d'un composé I selon la revendication 1 pour la préparation d'un médicament pour le traitement ou la prophylaxie de l'infarctus du myocarde.

8. Utilisation d'un composé I selon la revendication 1 pour la préparation d'un médicament pour le traitement ou la prophylaxie de l'angine de poitrine.

9. Utilisation d'un composé I selon la revendication 1 pour la préparation d'un médicament pour le traitement ou la prophylaxie d'états ischémiques du coeur.

10. Utilisation d'un composé I selon la revendication 1 pour la préparation d'un médicament pour le traitement ou la prophylaxie d'états ischémiques du système nerveux périphérique et central et de l'attaque.

11. Utilisation d'un composé I selon la réaction 1 pour la préparation d'un médicament pour le traitement ou la prophylaxie d'états ischémiques d'organes périphériques et de membres.

12. Utilisation d'un composé I selon la revendication 1 pour la préparation d'un médicament pour le traitement des états de choc.

13. Utilisation d'un composé I selon la revendication 1 pour la préparation d'un médicament destiné à être utilisé dans les opérations chirurgicales et les transplantations d'organes.

14. Utilisation d'un composé I selon la revendication 1 pour la préparation d'un médicament pour la conservation et le stockage de greffes pour des opérations chirurgicales.

15. Utilisation d'un composé I selon la revendication 1 pour la préparation d'un médicament pour le traitement des maladies dans lesquelles la prolifération cellulaire constitue une cause primaire ou secondaire, et donc son utilisation pour la préparation d'un agent antiathérosclérotique, d'un agent contre les complications diabétiques tardives, les maladies cancéreuses, les maladies fibreuses comme la fibrose pulmonaire, la fibrose hépatique ou la fibrose rénale, l'hyperplasie de la prostate.

16. Utilisation d'un composé I selon la revendication 1 pour la préparation d'un médicament pour le traitement ou la prophylaxie de troubles du métabolisme des graisses.

17. Médicament **caractérisé par** une teneur efficace d'un composé de formule I selon les revendications 1 à 4.
